(19) Europäisches Patentamt
European Patent Office
Office européen des brevets

(11) Veröffentlichungsnummer: **0 090 239**
**B1**

(12) # EUROPÄISCHE PATENTSCHRIFT

(45) Veröffentlichungstag der Patentschrift: **15.01.86**

(51) Int. Cl.⁴: **C 07 D 303/04**, C 07 D 301/14

(21) Anmeldenummer: **83102477.3**

(22) Anmeldetag: **14.03.83**

(54) **Verfahren zur Herstellung von 2,2-Bis-(3-cyclohexenyl)-propan-diepoxid.**

(30) Priorität: **26.03.82 DE 3211305**

(43) Veröffentlichungstag der Anmeldung: **06.10.83 Patentblatt 83/40**

(45) Bekanntmachung des Hinweises auf die Patenterteilung: **15.01.86 Patentblatt 86/3**

(84) Benannte Vertragsstaaten: **DE FR GB**

(56) Entgegenhaltungen:
**DE-B-1 015 782**
**DE-B-1 099 733**
**GB-A-1 497 970**
**US-A-2 745 848**

**Die Akte enthält technische Angaben, die nach dem Eingang der Anmeldung eingereicht wurden und die nicht in dieser Patentschrift enthalten sind.**

(73) Patentinhaber: **BAYER AG, Konzernverwaltung RP Patentabteilung, D-5090 Leverkusen 1 Bayerwerk (DE)**

(72) Erfinder: **Rauleder, Gebhard, Dr., Mozartstrasse 20, D-5657 Haan (DE)**
Erfinder: **Waldmann, Helmut, Dr., Henry-T.-von Boettinger Strasse 15, D-5090 Leverkusen 1 (DE)**

**EP 0 090 239 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1986

**Beschreibung**

Die vorliegende Erfindung betrifft ein Verfahren zur Herstellung von 2,2-Bis-(3-cyclohexenyl)-propan-diepoxid durch Umsetzung von 2,2-Bis-(3-cyclohexenyl)-propan mit Perpropionsäure.

2,2-Bis-(3-cyclohexenyl)-propan-diepoxid findet z.B. Verwendung als Kettenverlängerer für Polyamide (s. DE-B-1 099 733), als Stabilisator für halogenierte Kohlenwasserstoffe (s. JP-A-17964/75) und als Polymerisationskomponente zur Herstellung von Zahnfüllungen (s. DE-B-2 406 557).

Es ist grundsätzlich bekannt, 2,2-Bis-(3-cyclohexenyl)-propan-diepoxid durch Umsetzung von 2,2-Bis-(3-cyclohexenyl)-propan mit einer Percarbonsäure im Sinne einer Prileschajew-Reaktion entsprechend folgender Reaktionsgleichung herzustellen:

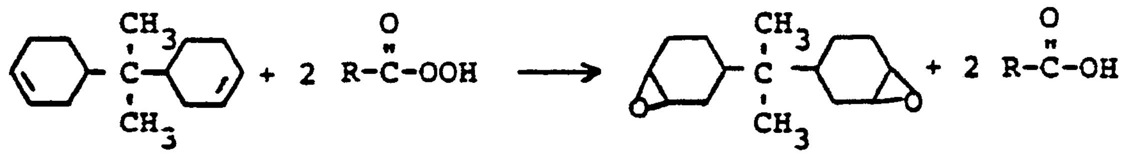

Die Herstellung von 2,2-Bis-(3-cyclohexenyl)-propan-diepoxid nach dieser Reaktion ist bisher jedoch nicht auf befriedigende Weise möglich. So wird in der DE-B-1 099 733 im Beispiel A die Epoxidation von 2,2-Bis-(3-cyclohexenyl)-propan mit Peressigsäure beschrieben. Obwohl die Reaktion bei milden Temperaturen von 0 bis 28°C durchgeführt wird, wird 2,2-Bis-(3-cyclohexenyl)-propan-diepoxid nur in einer Ausbeute von etwa 33 % erhalten.

Eine wesentliche Verbesserung erbrachte die Durchführung dieser Epoxidation in Gegenwart von Puffersalzen. So werden gemäß der DE-B-1 015 782, Beispiel 4, 51 g 2,2-Bis-(3-cyclohexenyl)-propan in Methylenchlorid mit 80 %iger Peressigsäure unter Zusatz von 60 g wasserfreiem Natriumacetat umgesetzt. Nach einer Reaktionszeit von 2 Tagen bei Raumtemperatur wird mit Wasser ausgeschüttelt, mit Sodalösung neutral gewaschen und nach dem Trocknen über wasserfreiem Kaliumcarbonat und Abdestillieren des Lösungsmittels durch Destillation 2,2-Bis-(3-cyclohexenyl)-propan-diepoxid erhalten. Die Ausbeute an 2,2-Bis-(3-cyclohexenyl)-propan-diepoxid beträgt 95 %.

Für eine technische Anwendung sind Reaktionszeiten von 2 Tagen unwirtschaftlich. Weiterhin besteht ein großer Nachteil dieses Verfahrens darin, daß große Mengen an salzhaltigen Abwässern anfallen (es ist gewichtsmäßig mehr Natriumacetat als 2,2-Bis-(3-cyclohexenyl)-propan einzusetzen), die für eine technische Anwendung prohibitiv sind.

Wird nur ein Viertel der angegebenen Menge an Natriumacetat verwendet, so sinkt die Ausbeute an 2,2-Bis-(3-cyclohexenyl)-propan-diepoxid unter sonst gleichen Bedingungen auf 47 % (s. DE-B-1 015 782, Beispiel 4). Ohne Zusatz von Natriumacetat wird lediglich ein undestillierbares Harz erhalten (s. DE-B-1 015 782, Beispiel 4).

Schließlich ist aus der US-A-2, 745, 848 ein Verfahren zur Herstellung von Cyclohexenoxid bekannt, bei dem man Perpropionsäure und ein organisches Lösungsmittel einsetzt. Auch hierbei muß der pH-Wert durch Zusätze genau kontrolliert werden.

Es wurde nun ein Verfahren zur Herstellung von 2,2-Bis-(3-cyclohexenyl)-propan-diepoxid durch Umsetzung von 2,2-Bis-(3-cyclohexenyl)-propan mit Perpropionsäure bei 0 bis 90°C und in Gegenwart eines organischen Lösungsmittels gefunden, das dadurch gekennzeichnet ist, daß man 2,2-Bis-(3-cyclohexenyl)-propan mit einer Lösung von Perpropionsäure in einem organischen Lösungsmittel umsetzt, wobei die Perpropionsäurelösung einen Wassergehalt von unter 2 Gew.-%, einen Gehalt an Wasserstoffperoxid von unter 0,5 Gew.-% und einen Gehalt an Mineralsäure von unter 50 ppm aufweist und das Molverhältnis von Perpropionsäure zu 2,2-Bis-(3-cyclohexenyl)-propan 1,5:1 bis 2,5:1 beträgt und keine Puffersalze zusetzt.

Das erfindungsgemäß einzusetzende Ausgangsprodukt 2,2-Bis-(3-cyclohexenyl)-propan ist eine bekannte Verbindung, die beispielsweise gemäß DE-B-1 099 733, Beispiel A, oder auf sonstige Weise hergestellt werden kann.

Als organisches Lösungsmittel für die Perpropionsäure können in der erfindungsgemäßen Umsetzung beispielsweise die verschiedensten unsubstituierten oder substituierten Kohlenwasserstoffe verwendet werden, die unter den Reaktionsbedingungen flüssig sind und unerwünschte Nebenreaktionen nicht oder nur in ganz untergeordnetem Maße eingehen. Solche Kohlenwasserstoffe sind z.B. aliphatische und cycloaliphatische Kohlenwasserstoffe, wie Hexan, Heptan, Octan, 2-Ethyl-hexan, Decan, Dodecan, Cyclohexan, Methylcyclopentan und Petrolether; aromatische Kohlenwasserstoffe wie Benzol, Nitrobenzol, Toluol, Ethylbenzol, Cumol, Diisopropylbenzol, Xylol, Chlorbenzol und Dichlorbenzol; sauerstoffhaltige Kohlenwasserstoffe wie Ether und Ester, z.B. Diethylether, Diisopropylether, Dibutylether, Tetrahydrofuran, Dioxan, Essigsäureethylester, Essigsäuremethylester, Essigsäurepropylester, Essigsäurebutylester, Propionsäuremethylester, Propionsäureethylester, Propionsäurepropylester, Buttersäuremethylester, Buttersäureethylester, Buttersäurepropylester, Buttersäurebutylester, Benzoesäuremethylester und Benzoesäureethylester; chlorierte Kohlenwasserstoffe wie Methylenchlorid, Chloroform, Tetrachlorkohlenstoff, 1-Chlorethan, 1,2-Dichlorethan, 1,1-Dichlorethan, 1,1,2,2-Tetrachlorethan, 1-Chlorpropan, 2-Chlorpropan, 1,2-Dichlorpropan, 1,3-Dichlorpropan, 2,3-Dichlorpropan, 1,2,3-Trichlorpropan, 1,1,2,3-Tetrachlorpropan, Butylchlorid, 1,2-Dichlorbutan, 1,4-Dichlorbutan, 2,3-Dichlorbutan, 1,3-Dichlorbutan, 1,2,3,4-Tetrachlorbutan, tert.-Butylchlorid, Amylchlorid, 1,2-Dichlorpentan,

1,5-Dichlorpentan, 1,2,3,4-Tetrachlorpentan, Cyclopentylchlorid, 1,2-Dichlorcyclopentylchlorid, Hexylchlorid, 1,2-Dichlorhexan, 1,6-Dichlorhexan, 1,2,3,4-Tetrachlorhexan, 1,2,5,6-Tetrachlorhexan, Cyclohexylchlorid, Chlorbenzol, Dichlorbenzol, Heptylchlorid, 1,2-Dichlorheptan, 1,2,3,4-Tetrachlorheptan, Cycloheptylchlorid, Octylchlorid, 1,2-Dichloroctan, 1,2,3,4-Tetrachloroctan und Cyclooctylchlorid.

Bevorzugte Lösungsmittel sind von den chlorierten Kohlenwasserstoffen Methylenchlorid, Chloroform, Tetrachlorkohlenstoff und 1,2-Dichlorpropan, von den aromatischen Kohlenwasserstoffen Benzol, Nitrobenzol, Toluol und Dichlorbenzol, von den aliphatischen und cycloaliphatischen Kohlenwasserstoffen 2-Ethylhexan, Cyclohexan und Methylcyclopentan und von den sauerstoffhaltigen Kohlenwasserstoffen Tetrahydrofuran, Propionsäureethylester und Benzoesäureethylester.

Besonders bevorzugte Lösungsmittel sind von den chlorierten Kohlenwasserstoffen 1,2-Dichlorpropan und Tetrachlorkohlenstoff, von den aromatischen Kohlenwasserstoffen Benzol und Dichlorbenzol, von den aliphatischen und cycloaliphatischen Kohlenwasserstoffen Cyclohexan und von den sauerstoffhaltigen Kohlenwasserstoffen Propionsäureethylester und Benzoesäureethylester.

Verwendet werden können auch Gemische der verschiedenen oben angegebenen organischen Lösungsmittel.

Die Perpropionsäure, gelöst in einem der genannten organischen Lösungsmittel, kann z.B. nach dem in der DE-A- 2 262 970 beschriebenen Verfahren hergestellt werden, bei dem wäßriges Wasserstoffperoxid mit der entsprechenden Carbonsäure in Gegenwart von Schwefelsäure umgesetzt und anschließend die entstandene Perpropionsäure mit dem organischen Lösungsmittel aus dem Reaktionsgemisch extrahiert wird. Gegebenenfalls kann die so erhaltene Perpropionsäurelösung in dem organischen Lösungsmittel noch weiter gereinigt werden, insbesondere um den Gehalt an Wasser, Wasserstoffperoxid und Schwefelsäure zu erniedrigen.

Die Lösungen, die organisches Lösungsmittel und Perpropionsäure enthalten, können beispielsweise 10 bis 30 Gew.-% Perpropionsäure, bezogen auf die Lösung, enthalten.

2,2-Bis-(3-cyclohexenyl)-propan kann als solches oder ebenfalls gelöst in einem oder mehreren der vorstehend genannten Lösungsmittel eingesetzt werden, wobei beliebig konzentrierte Lösungen zum Einsatz gelangen können. Vorzugsweise wird 2,2-Bis-(3-cyclohexenyl)-propan als solches eingesetzt und organische Lösungsmittel nur in Form der Perpropionsäurelösung zugegeben.

Das Molverhältnis von Perpropionsäure zu 2,2-Bis-(3-cyclohexenyl)-propan beträgt erfindungsgemäß 1,5:1 bis 2,5:1. Bevorzugt beträgt dieses Molverhältnis 1,7:1 bis 2,3:1. Besonders bevorzugt werden pro Mol 2,2-Bis-(3-cyclohexenyl)-propan 1,8 bis 2,2 Mol Perpropionsäure eingesetzt.

Der Wassergehalt der verwendeten Perpropionsäurelösung soll im allgemeinen möglichst niedrig sein und unter 2 Gew.-% betragen. Insbesondere geeignet sind beispielsweise Perpropionsäurelösungen mit einem Wassergehalt von bis zu 1 Gew.-%. Vorzugsweise verwendet man Perpropionsäurelösungen, die weniger als 0,5 Gew.-% Wasser enthalten. Besonders bevorzugt ist ein Wassergehalt von weniger als 0,1 Gew.-%.

Der Wasserstoffperoxidgehalt der verwendeten Perpropionsäurelösung soll im allgemeinen ebenfalls möglichst niedrig sein. Er kann bis zu 0,5 Gew.-%, bezogen auf die Perpropionsäurelösung, betragen. Vorteilhaft arbeitet man bei einem Gehalt von weniger als 0,35 Gew.-%. Besonders vorteilhaft ist es, die Umsetzung mit einer Perpropionsäurelösung durchzuführen, die einen Wasserstoffperoxidgehalt unter 0,2 Gew.-% aufweist.

Der Mineralsäuregehalt der verwendeten Perpropionsäurelösung soll auch möglichst niedrig sein und unterhalb 50 ppm liegen. Besonders vorteilhaft ist ein Mineralsäuregehalt von weniger als 10 ppm.

Die erfindungsgemäße Umsetzung wird im Temperaturbereich von 0 bis 90°C durchgeführt. Bevorzugt arbeitet man bei 10 bis 80°C, besonders bevorzugt bei 20 bis 70°C. In Sonderfällen können die angegebenen Temperaturen auch unter- oder überschritten werden.

Die erfindungsgemäße Umsetzung kann bei den verschiedensten Drucken durchgeführt werden. Im allgemeinen arbeitet man bei Normaldruck. Das Verfahren kann jedoch auch bei Unter- oder Überdruck durchgeführt werden.

Die Durchführung der erfindungsgemäßen Umsetzung kann diskontinuierlich oder kontinuierlich in den für Umsetzungen dieser Art üblichen Vorrichtungen, wie Rührwerkskesseln, Siedereaktoren, Röhrenreaktoren, Schlaufenreaktoren oder Schleifenreaktoren, erfolgen.

Als Werkstoffe für die Reaktionsäpparate zur Durchführung der erfindungsgemäßen Umsetzung können beispielsweise Glas, Edelstähle oder emailliertes Material verwendet werden.

Schwermetallionen im Reaktionsgemisch katalysieren die Zersetzung von Percarbonsäuren. Es ist deshalb vorteilhaft, der Perpropionsäurelösung Substanzen zuzusetzen, die Schwermetallionen durch Komplexbildung inaktivieren können. Substanzen solcher Art sind beispielsweise Gluconsäure, Ethylendiamintetraessigsäure, Natriumsilikat, Natriumpyrophosphat, Natriumhexamethaphosphat, Dinatriumdimethylpyrophosphat oder $Na_5$ (2-Ethyl-hexyl)$_5$ $(P_3O_{10})_2$ (vgl. DE-B- 1 056 596, Spalte 4, Zeilen 60 ff).

Die Reaktionswärme kann auf beliebige Weise abgeführt werden, beispielsweise durch innen- oder außenliegende Kühler. Zur Ableitung der Reaktionswärme kann die Umsetzung auch unter Rückfluß, z.B. in Siedereaktoren, durchgeführt werden.

2,2-Bis-(3-cyclohexenyl)-propan und die Perpropionsäurelösung können auf beliebige Weise zusammengebracht werden. Beispielsweise kann man beide Komponenten gleichzeitig oder nacheinander in beliebiger Reihenfolge in das Reaktionsgefäß einbringen. Bei diskontinuierlicher Arbeitsweise wird vorzugsweise 2,2-Bis-(3-cyclohexenyl)-propan vorgelegt und dann die Perpropionsäurelösung zugegeben. Die Reaktionstemperatur kann dabei vor oder nach der Zugabe der Perpropionsäurelösung eingestellt werden. Bei kontinuierlicher Arbeitsweise kann man die beiden Komponenten gemeinsam oder getrennt dem Reaktor

zuführen. Bei Verwendung mehrerer Reaktoren, die beispielsweise als Kaskade hintereinander geschaltet sein können, kann es vorteilhaft sein, 2,2-Bis-(3-cyclohexenyl)-propan nur in den ersten Reaktor einzubringen. Man kann die 2,2-Bis-(3-cyclohexenyl)-propan-Zugabe jedoch auch auf mehrere Reaktoren verteilen.

Es kann weiterhin von Vorteil sein, die benötigte Menge Perpropionsäure chargenweise zuzugeben. Dabei kann es von Vorteil sein monoepoxidiertes 2 2-Bis-(3-cyclohexenyl)-propan aus dem Reaktionsgemisch abzutrennen und einer erneuten Umsetzung mit Perpropionsäure zu unterwerfen. Bei der chargenweisen Zugabe der Perpropionsäure kann es auch von Vorteil sein, nach Zugabe einzelner Chargen die aus der Perpropionsäure entstandene Propionsäure aus dem Reaktionsgemisch zu entfernen, z.B. durch Extraktion mit Wasser oder durch Destillation.

Die nach Durchführung des erfindungsgemäßen Verfahrens erhaltenen Reaktionsgemische enthalten im allgemeinen das verwendete organische Lösungsmittel, die aus der Perpropionsäure entstandene Propionsäure und 2,2-Bis-(3-cyclohexenyl)-propan-diepoxid, wobei gegebenenfalls zusätzlich monoepoxidiertes und unumgesetztes 2,2-Bis-(3-cyclohexenyl)-propan, sowie gegebenenfalls geringe Mengen von hochsiedenden Nebenprodukten im Reaktionsgemisch vorhanden sein können.

Die Aufarbeitung des erhaltenen Reaktionsgemisches kann z.B. durch Destillation erfolgen. Dabei kann man so verfahren, daß man die einzelnen Komponenten in der Reihenfolge ihrer Siedepunkte abdestilliert. Dabei ist es von Vorteil, die Destillation im Vakuum und unter Verwendung von Verdampfern, die kurze Verweilzeiten und eine Verdampfung mit geringer thermischer Belastung des Reaktionsgemisches ermöglichen, durchzuführen. Wenn Perpropionsäure im molaren Überschuß eingesetzt worden ist, kann es u.U. von Vorteil sein, vor der destillativen Aufarbeitung dem Reaktionsgemisch ein Zusatzlösungsmittel zuzufügen, das zwischen Propionsäure und 2,2-Bis-(3-cyclohexenyl)-propan-diepoxid siedet. Dadurch erreicht man eine vollständige Abtrennung der Propionsäure bei relativ niedrigen Sumpftemperaturen, was hinsichtlich der Minimierung der Bildung von Nebenprodukten aus der Propionsäure und den epoxidierten Bestandteilen des Reaktionsgemisches von Vorteil sein kann.

Es kann u.U. weiterhin von Vorteil sein, insbesondere wenn die Perpropionsäure im Überschuß eingesetzt wurde, vor der destillativen Aufarbeitung aus dem Reaktionsgemisch die Propionsäure durch Extraktion, mit z.B. Wasser, zu entfernen. Aus der so erhaltenen wäßrigen Propionsäurelösung kann die Propionsäure wieder durch Extraktion mit einem geeigneten Lösungsmittel zurückgewonnen und gegebenenfalls zur Herstellung der Perpropionsäure in einem organischen Lösungsmittel verwendet werden.

Es kann vorteilhaft sein, dem Reaktionsgemisch vor oder während der Aufarbeitung, insbesondere bei destillativer Aufarbeitung, Stabilisatoren zuzusetzen, welche die Bildung von Hochsiedern und Polymerisaten verhindern bzw. minimieren.

Aus dem Reaktionsgemisch abgetrenntes nicht umgesetztes 2,2-Bis-(3-cyclohexenyl)-propan und 2,2-Bis-(3-cyclohexenyl)-propan-monoepoxid können erneut in das erfindungsgemäße Verfahren eingesetzt werden.

Das erfindungsgemäße Verfahren hat den Vorteil, daß danach 2,2-Bis-(3-cyclohexenyl)-propan-diepoxid in hohen Ausbeuten und ohne Zusatz von Puffersalzen zugänglich ist. Im Hinblick auf den eingangs geschilderten Stand der Technik ist es ausgesprochen überraschend, daß 2,2-Bis-(3-cyclohexenyl)-propan-diepoxid ohne Zusatz von Puffersalzen in hohen Ausbeuten erhalten werden kann.

**Beispiele**

**Beispiel 1**

In einen 5 1 Doppelwandkolben mit Rührer, Rückflußkühler und Tropftrichter wurden 612 g (3 Mol) 2,2-Bis-(3-cyclohexenyl) -propan vorgelegt und auf 60°C thermostatisiert. Unter Rühren wurden 5,7 Mol = 2565 g 20 %ige benzolische Perpropionsäurelösung (weniger als 0,1 Gew.-% Wasser, weniger als 0,2 Gew.-% Wasserstoffperoxid und weniger als 10 ppm Mineralsäure enthaltend) so schnell zugegeben, daß die Temperatur auf 60°C gehalten werden konnte. Nach dem Ende der Zugabe wurde noch 15 Minuten bei 60°C weitergerührt. Danach war der Perpropionsäureumsatz praktisch quantitativ. Die gaschromatographische Analyse ergab, daß sich 68,2 g (0,31 Mol) 2,2-Bis-(3-cyclohexenyl)-propan-monoepoxid und 614,4 g (2,56 Mol) 2,2-Bis-(3-cyclohexenyl)-propan-diepoxid gebildet hatten. Das entspricht einer Gesamtepoxidausbeute von 95,7 % der Theorie.

Nach Zugabe von 400 g Benzoesäureethylester wurde in einer mit einem Fallstromverdampfer betriebenen Füllkörperkolonne bei einem Druck von 200 mbar Benzol und Propionsäure als Kopfprodukt abgetrennt. Sie wurden in einer weiteren Kolonne in die reinen Komponenten aufgetrennt.

Anschließend wurde das von Benzol und Propionsäure befreite Gemisch bei vermindertem Druck fraktioniert. Nach Abdestillieren des noch verbliebenen Benzoesäureethylesters wurde bei einem Druck von 1,3 mbar 2,2-Bis-(3-cyclohexenyl)-propan-monoepoxid bei einer Kopftemperatur von 118°C und 2,2-Bis-(3-cyclohexenyl)-propan-diepoxid bei einer Kopftemperatur von 138 bis 140°C erhalten. Das erhaltene 2,2-Bis-(3-cyclohexenyl)-propan-monoepoxid wurde in einem weiteren Reaktionsansatz wieder eingesetzt.

**Beispiel 2**

In einer 5-stufigen Kesselkaskade aus Glas mit einem Gesamtvolumen von 1,5 l wurden stündlich bei einer Reaktionstemperatur von 50°C 945 g einer 20 gew.-%igen (2,1 Mol) Perpropionsäurelösung in Benzol und 204 g (1 Mol) 2,2 -Bis-(3-cyclohexenyl)-propan über getrennte Leitungen in den ersten Kessel der Kaskade eindosiert. Die Perpropionsäurelösung enthielt weniger als 0,1 Gew.-% Wasser, weniger als 0,2 Gew.-% Wasserstoffperoxid und weniger als 10 ppm Mineralsäure. Nach dem fünften Kessel resultierte ein Umsatz von 99,9 %, bezogen auf 2,2-Bis-(3-cyclohexenyl)-propan. Die gaschromatographische Analyse ergab, daß 2,2-Bis-(3-cyclohexenyl)-propan-monoepoxid mit 2,3 % Ausbeute der Theorie und 2,2-Bis-(3-cyclohexenyl)-propan-diepoxid mit 91,5 % Ausbeute der Theorie gebildet worden sind.

Das die Kaskade verlassende Reaktionsgemisch wurde in einem ersten Extraktor, einem pulsierten Siebbodenextraktor, mit der 1,5-fachen Gewichtsmenge Wasser im Gegenstrom extrahiert. Dabei erhielt man eine am Kopf des Extraktors abgezogene organische Phase mit einem Propionsäureanteil von unter 0,1 Gew.-%. Aus diesem Gemisch wurde in einer ersten Destillationskolonne bei einem Druck von 200 mbar und einer Kopftemperatur von 36 bis 37°C Benzol in einer zur Herstellung der eingangs genannten Perpropionsäurelösung geeigneten Reinheit abgetrennt. Aus dem Sumpf dieser Kolonne wurden anschließend bei einem Druck von 1,3 mbar, wie in Beispiel 1 beschrieben, die Epoxide erhalten. Die am unteren Ende des ersten Extraktors abgezogene wäßrige Phase, welche Propionsäure enthielt, wurde in einem zweiten Extraktor, ebenfalls einem pulsierten Siebbodenextraktor, mit der 1,5-fachen Gewichtsmenge Propionsäureethylester im Gegenstrom extrahiert. Das diesen Extraktor verlassende, weitgehend propionsäurefreie Wasser, wurde zur Extraktion des Reaktionsgemisches in den ersten Extraktor zurückgeführt. Die den zweiten Extraktor verlassende organische Phase, welche die Propionsäure und Propionsäureethylester enthielt, wurde in einer Destillationskolonne in ein Kopfprodukt, bestehend aus dem propionsäureethylester und in ein Sumpfprodukt aufgetrennt, das im wesentlichen aus Propionsäure bestand. Das Kopfprodukt dieser Destillationskolonne wurde in den zweiten Extraktor zur Extraktion der wäßrigen Phase aus dem ersten Extraktor zurückgeführt.

**Patentansprüche**

1) Verfahren zur Herstellung von 2,2-Bis-(3-cyclohexenyl)-propan-diepoxid durch Umsetzung von 2,2-Bis-(3-cyclohexenyl)-propan mit Perpropionsäure bei 0 bis 90°C und in Gegenwart eines organischen Lösungsmittels, dadurch gekennzeichnet, daß man 2,2-Bis-(3-cyclohexenyl)-propan mit einer Lösung von Perpropionsäure in einem organischen Lösungsmittel umsetzt, wobei die Perpropionsäurelösung einen Wassergehalt von unter 2 Gew.-%, einen Gehalt an Wasserstoffperoxid von unter 0,5 Gew.-% und einen Gehalt an Mineralsäure von unter 50 ppm aufweist, das Molverhältnis von Perpropionsäure zu 2,2-Bis-(3-cyclohexenyl)-propan 1,5:1 bis 2,5:1 beträgt und keine Puffersalze zusetzt.

2) Verfahren nach Anspruch 1, dadurch gekennzeichnet, daß man als organisches Lösungsmittel 1,2-Dichlorpropan, Tetrachlorkohlenstoff, Benzol, Dichlorbenzol, Cyclohexan, Propionsäureethylester oder Benzoesäureethylester einsetzt.

3) Verfahren nach Ansprüchen 1 und 2, dadurch gekennzeichnet, daß man eine 10 bis 30 gew.-%ige Lösung von Perpropionsäure in dem organischen Lösungsmittel einsetzt.

4) Verfahren nach Ansprüchen 1 bis 3, dadurch gekennzeichnet, daß man 2,2-Bis-(3-cyclohexenyl)-propan als solches einsetzt und organisches Lösungsmittel nur in Form der Perpropionsäurelösung zugibt.

5) Verfahren nach Ansprüchen 1 bis 4, dadurch gekennzeichnet, daß die Perpropionsäurelösung weniger als 1 gew.-% Wasser, weniger als 0,35 Gew.-% Wasserstoffperoxid und weniger als 10 ppm Mineralsäure enthält.

6) Verfahren nach Ansprüchen 1 bis 5, dadurch gekennzeichnet, daß man bei Temperaturen im Bereich 10 bis 80°C arbeitet.

7) Verfahren nach Ansprüchen 1 bis 6, dadurch gekennzeichnet, daß man die benötigte Menge Perpropionsäure chargenweise zugibt.

8) Verfahren nach Ansprüchen 1 bis 7, dadurch gekennzeichnet, daß man das erhaltene Reaktionsgemisch durch Destillation aufarbeitet, wobei man die einzelnen Komponenten in der Reihenfolge ihrer Siedepunkte abdestilliert.

9) Verfahren nach Anspruch 8, dadurch gekennzeichnet, daß man vor der destillativen Aufarbeitung dem Reaktionsgemisch ein Zusatzlösungsmittel zufügt, daß zwischen der aus der Perpropionsäure entstehenden Propionsäure und 2,2-Bis-(3-cyclohexenyl)-propan siedet.

10) Verfahren nach Ansprüchen 8 und 9, dadurch gekennzeichnet, daß man vor der destillativen Aufarbeitung aus dem Reaktionsgemisch die aus der Perpropionsäure entstandene Propionsäure durch Extraktion mit Wasser entfernt.

**Claims**

1. Process for the preparation of 2,2-bis-(3-cyclohexenyl)-propane diepoxide by reacting 2,2-bis-(3-cyclohexenyl)-propane with perpropionic acid at 0 to 90°C and in the presence of an organic solvent, characterised in that 2,2-bis-(3-cyclohexenyl)-propane is reacted with a solution of perpropionic acid in an organic solvent, the perpropionic acid solution having a water content of below 2% by weight, a content of hydrogen peroxide of below 0.5% by weight and a content of mineral acid of below 50 ppm, the molar ratio of perpropionic acid to 2,2-bis-(3-cyclohexenyl)-propane being 1.5:1 to 2.5:1 and no buffer salts being added.

2. Process according to Claim 1, characterised in that 1,2-dichloropropane, carbon tetrachloride, benzene, dichlorobenzene, cyclohexane, ethyl propionate or ethyl benzoate is used as the organic solvent.

3. Process according to Claims 1 and 2, characterised in that a 10 to 30% by weight solution of perpropionic acid in the organic solvent is used.

4. Process according to Claims 1 to 3, characterised in that 2,2-bis-(3-cyclohexenyl)-propane is used as such and organic solvent is added only in the form of the perpropionic acid solution.

5. Process according to Claims 1 to 4, characterised in that the perpropionic acid solution contains less than 1% by weight of water, less than 0.35% by weight of hydrogen peroxide and less than 10 ppm of mineral acid.

6. Process according to Claims 1 to 5, characterised in that it is carried out at temperatures in the range 10 to 80°C.

7. Process according to Claims 1 to 6, characterised in that the required quantity of perpropionic acid is added in batches.

8. Process according to Claims 1 to 7, characterised in that the reaction mixture obtained is worked up by distillation, the individual components being distilled off in the sequence of their boiling points.

9. Process according to Claim 8, characterised in that an additional solvent is added to the reaction mixture before the working up by distillation, which solvent boils between the propionic acid formed from the perpropionic acid and 2,2-bis-(3-cyclohexenyl)-propane.

10. Process according to Claims 8 and 9, characterised in that, before the working up by distillation, the propionic acid formed from the perpropionic acid is removed from the reaction mixture by extraction with water.

**Revendications**

1. Procédé de production de diépoxyde de 2,2-bis-(3-cyclohexényl)propane par réaction du 2,2-bis-(3-cyclohexényl)propane avec l'acide perpropionique à 0-90°C et en présence d'un solvant organique, caractérisé en ce qu'on fait réagir du 2,2-bis-(3-cyclohexényl)propane avec une solution d'acide perpropionique dans un solvant organique, la solution d'acide perpropionique présentant une teneur en eau inférieure à 2 % en poids, une teneur en peroxyde d'hydrogène inférieure à 0,5 % en poids et une teneur en acide minéral de moins de 50 ppm, le rapport molaire de l'acide perpropionique au 2,2-bis-(3-cyclohexényl)propane ayant une valeur de 1,5:1 à 2,5:1 et aucun sel tampon n'étant ajouté.

2. Procédé suivant la revendication 1, caractérisé en ce qu'on utilise comme solvant organique le 1,2-dichloropropane, le tétrachlorure de carbone, le benzène, le dichlorobenzène, le cyclohexane, l'ester éthylique de l'acide propionique ou l'ester éthylique de l'acide benzoïque.

3. Procédé suivant les revendications 1 et 2, caractérisé en ce qu'on utilise une solution à 10-30 % en poids d'acide perpropionique dans le solvant organique.

4. Procédé suivant les revendications 1 à 3, caractérisé en ce qu'on utilise le 2,2-bis-(3-cyclohexényl)propane tel quel et on n'ajoute un solvant organique que sous la forme de la solution d'acide perpropionique.

5. Procédé suivant les revendications 1 à 4, caractérisé en ce que la solution d'acide perpropionique contient moins de 1 % en poids d'eau, moins de 0,35 % en poids de peroxyde d'hydrogène et moins de 10 ppm d'acide minéral.

6. Procédé suivant les revendications 1 à 5, caractérisé en ce qu'on opère à des températures comprises dans l'intervalle de 10 à 80°C.

7. Procédé suivant les revendications 1 à 6, caractérisé en ce qu'on ajoute par portions la quantité nécessaire d'acide perpropionique.

8. Procédé suivant les revendications 1 à 7, caractérisé en ce qu'on traite par distillation le mélange réactionnel obtenu, en chassant par distillation les composants individuels dans l'ordre de leurs points d'ébullition.

9. Procédé suivant la revendication 8, caractérisé en ce qu'avant le traitement par distillation, on ajoute au mélange réactionnel un solvant supplémentaire, qui bout entre l'acide propionique formé à partir de l'acide perpropionique et le 2,2-bis-(3-cyclohexényl)propane.

10. Procédé suivant les revendications 8 et 9, caractérisé en ce que l'acide propionique formé à partir de l'acide perpropionique est éliminé du mélange réactionnel par extraction à l'eau avant le traitement par distillation.